(19) **Europäisches Patentamt**
**European Patent Office**
**Office européen des brevets**

(11) **EP 1 698 360 B1**

(12) **EUROPÄISCHE PATENTSCHRIFT**

(45) Veröffentlichungstag und Bekanntmachung des Hinweises auf die Patenterteilung:
**14.01.2009 Patentblatt 2009/03**

(51) Int Cl.:
**A61M 1/16** *(2006.01)*

(21) Anmeldenummer: **05004881.8**

(22) Anmeldetag: **05.03.2005**

(54) **Dialysemaschine mit einer Einrichtung zur Bestimmung der Dialysedosis**

Dialysisapparatus with a device for determining the dialysis dosage

Machine de dialyse avec un dispositif pour déterminer la dose de dialyse

(84) Benannte Vertragsstaaten:
**AT BE BG CH CY CZ DE DK EE ES FI FR GB GR HU IE IS IT LI LT LU MC NL PL PT RO SE SI SK TR**

(43) Veröffentlichungstag der Anmeldung:
**06.09.2006 Patentblatt 2006/36**

(73) Patentinhaber: **B. Braun Avitum AG**
**49215 Glandorf (DE)**

(72) Erfinder:
• **Dolgos, Sandor**
**2000 Szentendre (HU)**
• **Kinoranyi, Gabor**
**1045 Budapest (HU)**

• **Szamko, Péter**
**2131 Göd (HU)**
• **Moll, Stefan**
**34212 Melsungen (DE)**
• **Sauerbach, Dieter**
**58313 Herdecke (DE)**

(74) Vertreter: **von Kreisler Selting Werner**
**Patentanwälte**
**P.O. Box 10 22 41**
**50462 Köln (DE)**

(56) Entgegenhaltungen:
**EP-A- 1 062 960        US-A1- 2001 004 523**
**US-B1- 6 284 141**

**Beschreibung**

Die Erfindung betrifft eine Dialysemaschine mit einem Dialysator, der an einen Blutkreislauf und einen Dialysatkreislauf angeschlossen ist, wobei im Blutkreislauf ein vorbestimmter Blutfluss und im Dialysatkreislauf ein vorbestimmbarer Dialysatfluss einstellbar ist, und mit einer Einrichtung zur Bestimmung der Dialysedosis Kt/V anhand der Clearance K des Dialysators, des patientenspezifischen Schadstoffverteilungsvolumens V und der Behandlungsdauer t.

Die Dialysedosis Kt/V stellt heute in der Dialyse den wichtigsten Parameter zur Überwachung der Dialysequalität dar. Als adäquater Wert für das langfristige Wohlbefinden des Patienten wird eine Dialysedosis Kt/V von ≥ 1,3 am Ende der Therapie angesehen. Vielfach gilt dieser Wert bereits als Zielgröße für die Therapie. Der zeitabhängige Wert Kt/V sollte während der Therapie laufend ermittelt werden. Er steigt durch seine Summierung kontinuierlich an, so dass er die erbrachte Dialysedosis angibt. Kt/V kann für verschiedene harnpflichtige Stoffe ermittelt werden. In der Praxis wird der Wert für Harnstoff benutzt, kann jedoch auch für andere im Blut enthaltene Schadstoffe angewandt werden. Die im Folgenden beschriebenen Berechnungen können in der Dialysemaschine oder mit den Daten der Maschine auf einem externen Netzwerk durchgeführt werden.

Im Folgenden wird zunächst die Dialysedosis Kt/V erläutert:

**K** ist die Clearance des Dialysators, also die Eigenschaft des Filters, unter bestimmten Bedingungen ein Teil des Blutstromes vollständig zu reinigen. Die Größe K ist vom Typ des jeweiligen Dialysators abhängig. Ihre Ermittlung wird später noch erläutert.

**t** ist die Zeit, also die laufende Therapiezeit.

**V** ist das patientenspezifische Harnstoffverteilungsvolumen. V ändert sich während der Therapie nur unwesentlich. Es gibt verschiedene Modell-Bildungen, um V zu ermitteln, z.B. die Watson-Formel oder die Grundformel. In alle diese Formeln gehen Patientenparameter ein, wie z.B. das Patiententrockengewicht, die Patientengröße, Alter und Geschlecht.

Die Werte t und V können auf einfache Weise bestimmt oder berechnet werden. Kritisch ist die Ermittlung des Wertes K. Dieser ist von dem gewählten Dialysator abhängig sowie von verschiedenen Geräteparametern und vom Behandlungsverlauf. Die korrekte Ermittlung von K während der gesamten Therapie hat großen Einfluss auf die Ermittlung der Dialysedosis.

Es gibt Dialysemaschinen, bei denen die Clearance K während der Behandlung gemessen wird. Diese Technik beruht auf der Äquivalenz der Diffusionskoeffizienten des Natriumions und des Harnstoffmoleküls. Im Dialysatkreislauf wird eingangsseitig und ausgangsseitig des Dialysators die Leitfähigkeit gemessen und daraus die Harnstoff-Clearance ermittelt. Derartige Messungen werden in größeren zeitlichen Abständen durchgeführt. Bei der Änderung von Behandlungsparametern wie z.B. der Größe des Blutflusses, der Größe des Dialysatflusses oder der Ultrafiltrationsmenge vergeht eine gewisse Zeit bis im Rahmen der nächsten Messung der aktuelle Wert K ermittelt wird und Kt/V in einer Grafik darstellbar ist. Eine Prognose des unter den aktuellen Einstellungen erreichbaren Endwertes Kt/V ist nicht möglich.

In US 6,284,141 B1 ist eine Dialysemaschine beschrieben, bei der die Dialysedosis durch Messung ermittelt wird. Ein bestimmter zeitlicher Verlauf der Effizienz der Dialysebehandlung wird als Grenzkurve in Form einer Profilkurve vorgegeben. Die tatsächliche Dialysedosis wird laufend gemessen und über der Grenzkurve gehalten.

Der Erfindung liegt die Aufgabe zugrunde, eine Dialysemaschine zu schaffen, die imstande ist, Änderungen der Dialysedosis unter Berücksichtigung von Änderungen von Maschinenparametern vorherzusagen, um qualifizierte Aussagen über die weitere Dauer und Qualität der Dialyse zu liefern.

Die erfindungsgemäße Dialysemaschine ist durch den Patentanspruch 1 definiert. Hiernach wird die Clearance K anhand eines mathematischen Modells des Dialysators ermittelt, dem als Eingangsgrößen eingestellte Maschinenparameter der Dialysemaschine zugeführt werden, wobei die Maschinenparameter die Größe des Blutflusses, die Größe des Dialysatflusses und des Ultrafiltrationsflusses umfassen. Anhand der so ermittelten Clearance wird die Dialysedosis vorausberechnet. Das mathematische Modell kann parallel zu einer tatsächlichen Messung Verwendung finden oder unabhängig von dieser angewendet werden. Es ermöglicht die Anzeige eines prognostizierten Endwertes Kt/V. Diese Anzeige ist jederzeit aktuell verfügbar. Auf diese Weise kann für unterschiedliche Maschineneinstellungen mit variierenden Maschinenparametern jeweils die Dialysedosis vorausbestimmt werden ohne zunächst die Auswirkungen der veränderten Maschinenparameter abwarten zu müssen.

Die Erfindung bietet den Vorteil, dass der Wert der Clearance K jederzeit und ohne vorherige Messungen verfügbar ist, so dass unter Benutzung dieses Wertes die Dialysedosis einer vorgesehenen Behandlung bestimmt werden kann. Dabei kann der Endwert der Dialysedosis, der sich bei Behandlungsende ergibt, leicht bestimmt werden. Dies ermöglicht es, festzustellen, ob der Endwert einem Zielwert entspricht. Andererseits kann auf einfache Weise festgestellt werden, um welches Maß die Behandlungsdauer verlängert oder verkürzt werden müsste, um den Endwert auf einen gewünschten Zielwert zu bringen. Schließlich ist es auch möglich, an der Maschine die Maschinenparameter zu verändern, um sofort ermitteln zu können, welche Auswirkungen diese Veränderung auf die Dialysedosis hat.

Das mathematische Modell zur Ermittlung der Clearance K des Dialysators kann eine Formel enthalten. Vorzugsweise ist dieses mathematische Modell in Form einer Tabelle hinterlegt, die für unterschiedliche Flüsse von Blut, Dialysat und Ultrafiltrat die betreffenden Werte von K angibt. Solche Tabellen sind herstellerseitig verfügbar. Sie können aber auch von der Dialysemaschine durch Messungen, die von Zeit zu Zeit durchgeführt werden, erstellt werden. Werte, die der Tabelle nicht direkt entnommen werden können, können durch Interpolation ermittelt werden. In der Dialysemaschine werden die entscheidenden Kenndaten des betreffenden Typs von Dialysator gespeichert, so dass daraus die Clearance K für unterschiedliche Maschinenparameter wie Blutfluss und Dialysatfluss abrufbar ist.

Die Maschinenparameter müssen nicht unbedingt durch unmittelbare Einstellung durch den Benutzer vorgegeben werden, sondern sie können auch von der Steuereinrichtung anhand anderer Benutzervorgaben errechnet werden. So kann beispielsweise vorgesehen sein, dass die Einrichtung zur Bestimmung der Dialysedosis aus einem vorgegebenen Ultrafiltrationsvolumen oder vorgegebenen Profilform den Ultrafiltrationsfluss errechnet. Auf dieser Grundlage kann ein Ultrafiltrationsprofil vorgegeben werden, bei dem sich der Ultrafiltrationsfluss nach einem zeitlichen Programm verändert. Für eine derartige komplexe Dialysebehandlung kann die Dialysedosis in Abhängigkeit von der jeweiligen Dialysezeit berechnet werden.

Das mathematische Modell kann für jeden Dialysatortyp die Abhängigkeit einer Clearance unter Laborbedingungen $K_L$ von Maschinenparametern enthalten. Die Clearance K eines Dialysators in ml/min ändert sich während der Therapie häufig und ist von dem Dialysatortyp, Geräteparametern und vom Behandlungsverlauf abhängig. Die korrekte Ermittlung von K während der gesamten Therapie hat großen Einfluss auf die Ermittlung des korrekten Kt/V.

Gemäß einer bevorzugten Ausgestaltung der Erfindung ist vorgesehen, dass das mathematische Modell einen Korrekturfaktor C(t) bildet, der die Verminderung der Filterleistung über die Therapiezeit angibt, wobei die Clearance K = $K_L$ * C(t) ist. Dadurch kann die Änderung der Clearance während der Therapie berücksichtigt werden.

Im Rahmen der Erfindung kann ein Zielwert für die Dialysedosis einstellbar sein, wobei ein Alarmsignal erzeugt wird, wenn die vorausberechnete Dialysedosis von dem eingestellten Zielwert abweicht. Auf diese Weise erkennt der Benutzer, ob der beabsichtigte Zielwert tatsächlich erreicht wird. Die Abweichung von dem eingestellten Zielwert kann als mögliche Verkürzung oder Verlängerung der eingestellten Therapiezeit angezeigt werden. Der Betreiber des Dialysegeräts erhält dann zugleich die Information, wie die Therapiezeit zu verändern ist, damit der Zielwert doch noch erreicht wird.

Im Folgenden wird unter Bezugnahme auf die Zeichnung ein Ausführungsbeispiel der Erfindung näher erläutert.

Es zeigen:

Figur 1    eine schematische Darstellung der Kreisläufe einer Dialysemaschine mit einer Einrichtung zur Steuerung der wichtigsten Komponenten,

Figur 2    ein Diagramm des zeitlichen Verlaufs der Dialysedosis während einer Dialysebehandlung, wobei der tatsächliche Verlauf und der prognostizierte Verlauf dargestellt sind, und

Figur 3    eine Darstellung eines anderen Therapieverlaufs mit prognostizierten Werten, wie sie von der Dialysemaschine angezeigt wird.

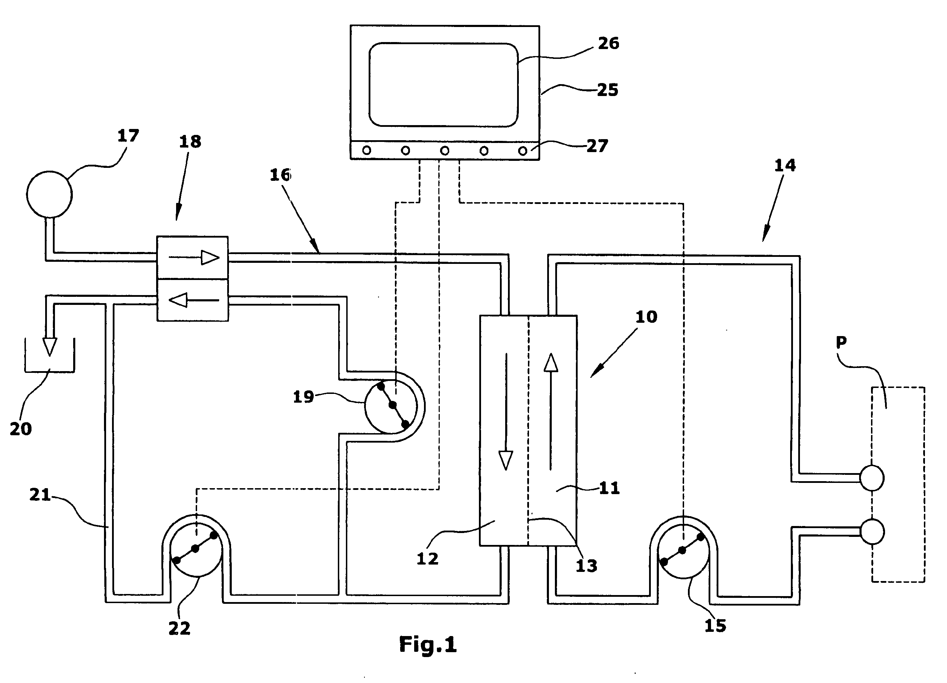

In Figur 1 ist das Kreislaufsystem einer Dialysemaschine schematisch dargestellt. Die Dialysemaschine weist einen Dialysator 10 auf mit einer ersten Kammer 11 und einer zweiten Kammer 12, die durch eine Dialysemembran 13 voneinander getrennt sind. Die erste Kammer 11 ist eine Blutkammer, die Bestandteil eines Blutkreislaufs 14 ist. Der Blutkreislauf führt von dem Körper eines Patienten P über eine Blutpumpe 15 durch die Kammer 11 hindurch zurück zum Patienten. Die zweite Kammer 12 ist Bestandteil eines Dialysatkreislaufs 16, der durch die zweite Kammer 12 führt. Der Dialysatkreislauf 16 führt von einer Dialysatquelle 17, welche Dialysierflüssigkeit liefert, durch eine Bilanziereinrichtung 18 zu der zweiten Kammer 12 und von dort über eine Dialysatpumpe 19 zu der Bilanziereinrichtung 18 und dann in einen Ablauf 20. Die Bilanziereinrichtung 18 bewirkt, dass die abgeführte Flüssigkeitsmenge gleich der zugeführten Flüssigkeitsmenge ist. Hinter dem Dialysator 10 zweigt eine Bypassleitung 21 ab, die eine weitere Pumpe 22 enthält und direkt in den Ablauf 20 führt. Die Bypassleitung 21 ist eine Ultrafiltrationsleitung. Durch Einstellung des Fördervolumens der Pumpe 22 kann das Ultrafiltrationsvolumen bestimmt werden, also dasjenige Volumen, das durch die Membran 13 des Dialysators hindurch zusätzlich in den Dialysatkreislauf 16 übertragen wird.

Alle Pumpen 15, 19 und 22 sind volumetrische Pumpen oder Verdrängerpumpen, bei denen das Fördervolumen zur Pumpengeschwindigkeit proportional ist. Die Pumpengeschwindigkeiten werden von einem Steuergerät 25 gesteuert, das eine Anzeigevorrichtung 26 in Form eines Bildschirms aufweist und eine Eingabevorrichtung 27 mit mehreren Tasten. Der Bildschirm 26 ist ein Touchscreen. Er bildet die Benutzerschnittstelle zum Einstellen und Ändern von Maschinenparametern und zur Information des Benutzers über den Betrieb der Dialysemaschine.

Das Steuergerät 25 empfängt darüber hinaus Signale von zahlreichen Sensoren, die hier nicht im Einzelnen dargestellt sind. Es steuert und überwacht die gesamte Dialysemaschine. Zu den Funktionen des Steuergerätes 25 gehört auch die Ermittlung der Dialysedosis Kt/V.

Das patientenspezifische Harnstoffverteilungsvolumen V wird aus den Werten Alter des Patienten, Körpergröße, Trockengewicht und Geschlecht des Patienten bestimmt. Diese Daten werden manuell in das Gerät eingegeben. Die Watson-Formel, nach der V berechnet wird, lautet

für männliche Patienten:

$$V \text{ (Liter)} = 2{,}447 - 0{,}09516 * \text{Alter (in Jahren)} + 0{,}1074 * \text{Körpergröße (cm)} + 0{,}3362 * \text{Trockengewicht (kg)};$$

für weibliche Patienten:

$$V \text{ (Liter)} = -2{,}097 + 0{,}1069 * \text{Körpergröße (cm)} + 0{,}2466 * \text{Trockenge-wicht (kg)}.$$

Die Größe V kann alternativ auch nach der folgenden Grundformel berechnet werden:

$$V \text{ (Liter)} = 0{,}58 * \text{Trockengewicht (kg)}.$$

Für die Eingabe der Patientendaten ist an dem Steuergerät 25 eine spezielle Bildschirmoberfläche vorgesehen, die unter Menüsteuerung aufgerufen werden kann.

Die Clearance K, die sich während der Therapie zeitabhängig ändert, errechnet sich nach der Formel:

$$K(t) = K_L * C(t).$$

K ist der sich mit der Therapiezeit t ändernde Wert der Clearance. $K_L$ ist die unter Laborbedingungen ermittelte Clearance des betreffenden Typs von Dialysator. C(t) ist ein Korrekturfaktor, der die Verminderung der Filterleistung $K_L$ über die Therapiezeit z.B. durch die Bildung einer Sekundärmembran beschreibt. $C \leq 1$, $C(t) = 1-(X_1 * t)$, wobei $X_1$ ein empirisch ermittelter Patienten/Filterfaktor ist.

$K_L$ ist eine Funktion von mehreren Geräteparametern:

$$K_L = f (Q_B; Q_D; Q_U; FT).$$

$Q_B$ ist der durch den Dialysator fließende Blutfluss
$Q_D$ ist der effiziente durch den Dialysator fließende Dialysatfluss
$Q_u$ ist der über die Membran fließende Ultrafiltrationsfluss
FT ist der Filtertyp.

$K_L$ wird in der Regel aus Tabellen oder dreidimensionalen Matrizen ermittelt, deren Werte unter Laborbedingungen gemessen wurden, und die in den Gebrauchsanweisungen der unterschiedlichen Dialysatoren angegeben sind.

In der nachfolgenden Tabelle 1 sind Beispiele für die Ermittlung von $K_L$ unter Vernachlässigung von $Q_u$ für einen Filtertyp angegeben:

## Tabelle 1

| $Q_D$ \ $Q_B$ | 100 ml/min | 200 ml/min | 300 ml/min | 400 ml/min | 500 ml/min |
|---|---|---|---|---|---|
| 300 ml/min | 70 | 140 | 210 | 290 | 400 |
| 500 ml/min | 80 | 160 | 240 | 335 | 430 |
| 800 ml/min | 85 | 170 | 255 | 355 | 460 |

In der Tabelle nicht angegebene Werte werden interpoliert.

Die Tabelle stellt das mathematische Modell des Dialysators dar. Außer der Möglichkeit für die Berechnung von K Laborwerte $K_L$ zu verwenden, gibt es die Möglichkeit, eine Tabelle wie die oben angegebene Tabelle 1 auch während der Therapie durch Messungen zu ermitteln oder nach der Therapie durch Laborwerte Kt/V zu optimieren. Damit kann eine Tabelle entstehen, die bereits in vivo Werte enthält und eine Korrektur mit dem Faktor C(t) kann entfallen.

Der Wert $Q_D$ ist in der Regel identisch mit dem in der Maschine eingestellten Dialysatfluss. Bei Therapien, bei denen aus dem Dialysatfluss noch Substitutionen gespeist werden, z.B. bei der Haemodiafiltration, sollte eine entsprechende Kompensation durchgeführt werden, um den effizienten Dialysatfluss zu erhalten.

Während bestimmter Therapieformen, wie sequenzieller Therapie, wird zwar ein eingestellter Dialysatlfluss von der Maschine gefördert, dieser fließt jedoch nicht durch den Dialysator und ist damit nicht relevant für die Bestimmung von K. Während sequenzieller Therapien und anderen Fällen, in denen der Dialysatfluss nicht durch den Dialysator fließt, ist $Q_D = 0$ und damit in der Regel auch K(t) = 0.

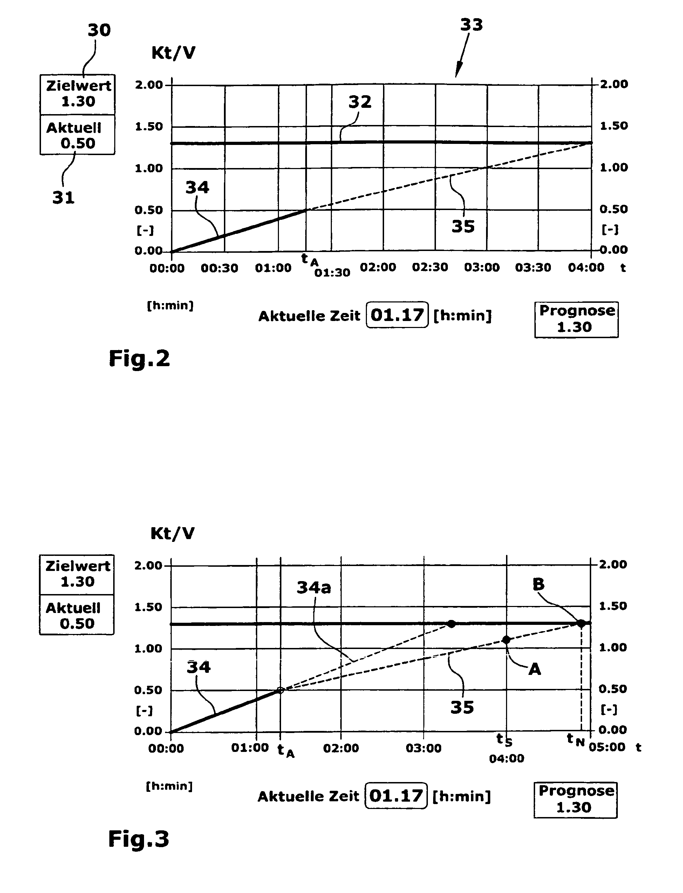

Figur 2 zeigt die Bildschirmoberfläche zur Darstellung der Dialysedosis Kt/V. Das Diagramm, das auf dem Bildschirm angezeigt wird, enthält eine Zielwertanzeige 30 und eine Anzeige 31 des aktuellen Wertes. Der manuell eingegebene Zielwert 32 wird als Balkenwert angezeigt. Das Diagramm 33 enthält längs der Abszisse die Therapiezeit t in Stunden und Minuten und längs der Ordinate die Dialysedosis Kt/V. Im vorliegenden Ausführungsbeispiel ist die Behandlung bis zum aktuellen Zeitwert $t_A$ bereits erfolgt. Daraus wurde eine Dialysedosis von 0,50 ermittelt. Die Kurve 34 gibt die abgelaufene Behandlung an und die gestrichelt dargestellte Kurve 35 den prognostizierten Behandlungsverlauf. Daraus ergibt sich bei dem dargestellten Beispiel, dass der Endwert nach vier Stunden mit dem geforderten Zielwert von 1,30 übereinstimmt.

Bei der Kurve nach Figur 2 erfolgt zum Zeitpunkt $t_A$ eine Veränderung der Behandlungsparameter, so dass die Kurven 34 und 35 unterschiedliche Steigungen haben.

Figur 3 zeigt ein Beispiel einer modifizierten Kurve. Zum Zeitpunkt $t_A$ geht der tatsächliche Verlauf 34 in den prognostizierten Verlauf 35 über. Dieser weicht von der linearen Fortsetzung 34a des Verlaufs 34 ab, weil zum Zeitpunkt $t_A$ eine Änderung von Maschinenparametern durchgeführt wurde. Der Verlauf zeigt, dass nach der eingestellten Therapiezeit $t_s$ von vier Stunden in Punkt A erst ein Wert Kt/V von 1,10 erreicht sein wird. Wenn die Behandlung in gleicher Weise fortgesetzt wird, wird der Zielwert von 1,30 nach einer Zeit $t_N$ in Punkt B erreicht.

Die Darstellung der Kurve Kt/V erfolgt in der Weise, dass die Punkte A und B auf dem Bildschirm sichtbar sind, so dass der Benutzer die Darstellung der eingestellten Therapiezeit und der tatsächlich notwendigen Therapiezeit sieht.

## Patentansprüche

1. Dialysemaschine mit einem Dialysator (10), der an einen Blutkreislauf (14) und an einen Dialysatkreislauf (16) angeschlossen ist, wobei im Blutkreislauf ein vorbestimmter Blutfluss und im Dialysatkreislauf ein vorbestimmter Dialysatfluss und Ultrafiltrationsfluss einstellbar ist, und mit einer Einrichtung (25) zur Bestimmung der Dialysedosis Kt/V anhand der Clearance K des Dialysators (10), des patientenspezifischen Schadstoffverteilungsvolumens V und der Behandlungsdauer t,

**dadurch gekennzeichnet,**

**dass** die Clearance K anhand eines mathematischen Modells des Dialysators (10) ermittelt wird, dem als Eingangsgrößen eingestellte Maschinenparameter der Dialysemaschine zugeführt werden, wobei die Maschinenparameter mindestens die Größe des Blutflusses $Q_B$ und die Größe des Dialysatflusses $Q_D$ umfassen, und dass die Dialysedosis anhand der so ermittelten Clearance vorausberechnet wird.

2. Dialysemaschine nach Anspruch 1, **dadurch gekennzeichnet, dass** die Einrichtung (25) zusätzlich die Größe des Ultrafiltrationsflusses mit einbezieht.

3. Dialysemaschine nach Anspruch 2, **dadurch gekennzeichnet, dass** die Einrichtung (25) die Dialysedosis bei einem eingestellten zeitlich variierenden Ultrafiltrationsprogramm aus den einzelnen Abschnitten dieses Programms errechnet.

4. Dialysemaschine nach einem der Ansprüche 1 bis 3, **dadurch gekennzeichnet, dass** das mathematische Modell für jeden Dialysatortyp die Abhängigkeit einer Clearance $K_L$ unter Laborbedingungen von Maschinenparametern enthält.

5. Dialysemaschine nach Anspruch 4, **dadurch gekennzeichnet, dass** das mathematische Modell einen Korrekturfaktor C(t) bildet, der die Verminderung der Filterleistung über die Therapiezeit angibt, wobei die Clearance $K = K_L * C(t)$ ist.

6. Dialysemaschine nach einem der Ansprüche 1 bis 3, **dadurch gekennzeichnet, dass** das mathematische Modell für K basierend auf Messungen während der Therapie angelegt wird.

7. Dialysemaschine nach einem der Ansprüche 1 bis 6, **dadurch gekennzeichnet, dass** ein Zielwert (B) für die Dialysedosis einstellbar ist und ein Alarmsignal erzeugt wird, wenn die vorausberechnete Dialysedosis (A) von dem eingestellten Zielwert abweicht.

8. Dialysemaschine nach Anspruch 7, **dadurch gekennzeichnet, dass** die Abweichung von dem eingestellten Zielwert (B) als mögliche Verkürzung oder Verlängerung der eingestellten Therapiezeit ($t_s$) angezeigt wird.

9. Dialysemaschine nach einem der Ansprüche 1 bis 8, **gekennzeichnet durch** ein Anzeigesystem, das den zeitlichen Verlauf der Dialysedosis bei den eingestellten Maschinenparametern anzeigt.

10. Dialysemaschine nach Anspruch 9, **dadurch gekennzeichnet, dass** das Anzeigesystem die Zeitachse derart ausdehnt, dass sowohl die eingestellte als auch die vorausberechnete Dialysedosis (A,B) auf ihr enthalten sind.

11. Dialysemaschine nach einem der Ansprüche 1 bis 10, **dadurch gekennzeichnet, dass** die vorausberechnete Dialysedosis unmittelbar nach Übernahme eingegebener Maschinenparameter für die Behandlung angezeigt wird.

12. Dialysemaschine nach einem der Ansprüche 1 bis 11, **dadurch gekennzeichnet, dass** die vorausberechnete Dialysedosis bereits nach Eingabe neuer Maschinenparameter und vor Übernahme für die Behandlung angezeigt wird.

13. Dialysemaschine nach einem der Ansprüche 1 bis 12, **dadurch gekennzeichnet, dass** die Dialysedosis, basierend auf aktuellen Daten der Dialysemaschine, auf einem mit der Maschine verbundenen externen Rechner vorausberechnet wird.

**Claims**

1. A dialysis machine comprising a dialyser (10) connected to a blood circuit (14) and a dialysate circuit (16), wherein a predetermined blood flow can be set in the blood circuit and a predetermined dialysate flow and ultrafiltration flow can be set in the dialysate circuit, and comprising means (25) for determining the dialysis dose Kt/V from the clearance K of the dialyser (10), the patient-specific toxic element distribution volume V and the duration of treatment t, **characterized in that**
   the clearance K is determined using a mathematical model of the dialyser (10) which is supplied with set machine parameters of the dialysis machine as input values, the machine parameters at least including the quantity of the blood flow $Q_B$ and the quantity of the dialysate flow $Q_D$, and that the dialysis dose is calculated in advance by means of the clearance thus determined.

2. The dialysis machine of claim 1, **characterized in that** the means (25) additionally includes the quantity of the ultrafiltration flow.

3. The dialysis machine of claim 2, **characterized in that**, for a set ultrafiltration program varying over time, the means (25) calculates the dialysis dose from the individual periods of this program.

4. The dialysis machine of one of claims 1 to 3, **characterized in that**, for each type of dialyser, the mathematical model includes the dependence of a clearance K, under laboratory conditions, on the machine parameters.

5. The dialysis machine of claim 4, **characterized in that** the mathematical model forms a correction factor C(t) indicating the reduction of the filtering performance over the duration of the therapy, where the clearance $K = K_L * C(t)$.

6. The dialysis machine of one of claims 1 to 3, **characterized in that** the mathematical model for K is established on the basis of measurements during the therapy.

7. The dialysis machine of one of claims 1 to 6, **characterized in that** a target value (B) may be set for the dialysis dose and an alarm signal is generated, when the pre-calculated dialysis dose (A) deviates from the set target value.

8. The dialysis machine of claim 7, **characterized in that** the deviation from the set target value (B) is indicated as a possible shortening or prolongation of the therapy time $(t_s)$.

9. The dialysis machine of one of claims 1 to 8, **characterized by** a display system displaying the temporal development of the dialysis dose for the machine parameters set.

10. The dialysis machine of claim 9, **characterized in that** the display system extends the time axis such that both the set dialysis dose and the pre-calculated dialysis dose (A, B) are included thereon.

11. The dialysis machine of one of claims 1 to 10, **characterized in that** the pre-calculated dialysis dose is indicated immediately after the acceptance of inputted machine parameters for the treatment.

12. The dialysis machine of one of claims 1 to 11, **characterized in that** the pre-calculated dialysis dose is displayed already after the input of new machine parameters and before their acceptance for the treatment.

13. The dialysis machine of one of claims 1 to 12, **characterized in that**, based on current data of the dialysis machine, the dialysis dose is pre-calculated on an external computer connected with the machine.

## Revendications

1. Machine de dialyse comprenant un dialyseur (10) connecté à un circuit de sang (14) et un circuit de dialysate (16), un écoulement de sang prédéterminé étant ajustable pour le circuit de sang et un écoulement prédéterminé de dialysate et d'ultrafiltration étant ajustable pour le circuit de dialysate, et comprenant un moyen (25) pour déterminer la dose de dialyse Kt/V selon la clairance K du dialyseur (10), le volume V de distribution des matières toxiques spécifique pour un patient et la durée de la thérapie t,
   **caractérisée en ce que**
   la clairance K est déterminée en utilisant un modèle mathématique du dialyseur (10) alimenté en paramètres de machine de la machine de dialyse comme paramètres d'entrée, les paramètres de machine comprenant au moins la quantité de l'écoulement de sang $Q_B$ et la quantité de l'écoulement du dialysate $Q_D$, et que la dose de dialyse et calculée en avance par la clairance tellement déterminée.

2. Machine de dialyse selon la revendication 1, **caractérisée en ce que** le moyen (25) aussi tient compte de la quantité de l'écoulement d'ultrafiltration.

3. Machine de dialyse selon la revendication 2, **caractérisée en ce qu'**en cas d'un programme d'ultrafiltration variable dans le temps étant ajusté, le moyen (25) calcule la dose de dialyse des phases individuelles de ce programme.

4. Machine de dialyse selon une des revendications 1 à 3, **caractérisée en ce que**, pour chaque type de dialyseur, le modèle mathématique comprend la dépendance d'une clairance K, sous conditions laboratoires, de paramètres de machine.

**5.** Machine de dialyse selon la revendication 4, **caractérisée en ce que** le modèle mathématique forme un facteur de correction C(t) indiquant la réduction de la capacité du filtre au cours du temps, la clairance étant K = $K_t$ * C(t).

**6.** Machine de dialyse selon une des revendications 1 à 3, **caractérisée en ce que** le modèle mathématique pour K est établi sur la base de mesures pendant la thérapie.

**7.** Machine de dialyse selon une des revendications 1 à 6, **caractérisée en ce qu'**une valeur de but (B) est ajustable pour la dose de dialyse et qu'un signal d'alerte est généré quand la dose de dialyse (A) calculée en avance diffère de la valeur de but ajustée.

**8.** Machine de dialyse selon la revendication 7, **caractérisée en ce que** la déviation de la valeur de but (B) ajustée est indiquée comme réduction ou prolongement possible de la durée ajustée de la thérapie ($t_s$).

**9.** Machine de dialyse selon une des revendications 1 à 8, **caractérisée par** un système d'affichage indiquant le développement temporel de la dose de dialyse avec les paramètres de machines ajustés.

**10.** Machine de dialyse selon la revendication 9, **caractérisée en ce que** le système d'affichage étend l'axe de temps de sorte que la dose de dialyse ajustée est inclut sur L#axe aussi bien que la dose de dialyse calculée en avance (A, B).

**11.** Machine de dialyse selon une des revendications 1 à 10, **caractérisée en ce que** la dose de dialyse calculée en avance est affichée directement après des paramètres de machine ont été acceptés pour la thérapie.

**12.** Machine de dialyse selon une des revendications 1 à 11, **caractérisée en ce que** la dose de dialyse calculée en avance est affichée déjà après avoir entré des nouveaux paramètres de machine et avant d'être accepter pour la thérapie.

**13.** Machine de dialyse selon une des revendications 1 à 12, **caractérisée en ce que** la dose de dialyse est calculée en avance, sur la base de données actuelles de la machine de dialyse, par un ordinateur externe lié avec la machine.

Fig.1

EP 1 698 360 B1

**30**

**33**

Kt/V

| Zielwert 1.30 |
| Aktuell 0.50 |

**31**

**32**

**34**

**35**

2.00 — 2.00

1.50 — 1.50

1.00 — 1.00

0.50 — 0.50

[-] [-]

0.00 — 0.00

00:00  00:30  01:00  $t_A$ 01:30  02:00  02:30  03:00  03:30  04:00  t

[h:min]

Aktuelle Zeit 01.17 [h:min]

| Prognose 1.30 |

## Fig.2

Kt/V

| Zielwert 1.30 |
| Aktuell 0.50 |

**34a**

**B**

**34**

**35**

**A**

2.00 — 2.00

1.50 — 1.50

1.00 — 1.00

0.50 — 0.50

[-] [-]

0.00 — 0.00

00:00  01:00  $t_A$  02:00  03:00  $t_S$ 04:00  $t_N$ 05:00  t

[h:min]

Aktuelle Zeit 01.17 [h:min]

| Prognose 1.30 |

## Fig.3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6284141 B1